(19) Europäisches Patentamt
European Patent Office
Office européen des brevets

(11) Veröffentlichungsnummer: **0 264 639 A1**

# (12) EUROPÄISCHE PATENTANMELDUNG

(21) Anmeldenummer: 87113750.1

(22) Anmeldetag: 19.09.87

(51) Int. Cl.⁴: **C07D 285/30**, C07D 285/24, C07D 417/12, A61K 31/54

(30) Priorität: 25.09.86 DE 3632544

(43) Veröffentlichungstag der Anmeldung: 27.04.88 Patentblatt 88/17

(84) Benannte Vertragsstaaten: AT BE CH DE ES FR GB GR IT LI LU NL SE

(71) Anmelder: BOEHRINGER INGELHEIM KG
D-6507 Ingelheim am Rhein(DE)

(84) BE CH DE ES FR GR IT LI LU NL SE AT

(71) Anmelder: Boehringer Ingelheim International G.m.b.H
D-6507 Ingelheim am Rhein(DE)

(84) GB

(72) Erfinder: Köppe, Herbert, Dr.
Neuweg 72
D-6507 Ingelheim am Rhein(DE)
Erfinder: Esser, Franz, Dr.
Posener Strasse 30
D-6507 Ingelheim am Rhein(DE)
Erfinder: Gaida, Wolfram, Dr.
Selztalstrasse 77 b
D-6507 Ingelheim am Rhein(DE)
Erfinder: Hoefke, Wolfgang, Dr.
Rosselstrasse 27
D-6200 Wiesbaden(DE)
Erfinder: Speck, Georg, Dr.
Rheinstrasse 13
D-6507 Ingelheim am Rhein(DE)

## (54) Neue Aryloxy-aminoalkane, ihre Herstellung und Verwendung.

(57) Verbindungen der Formel

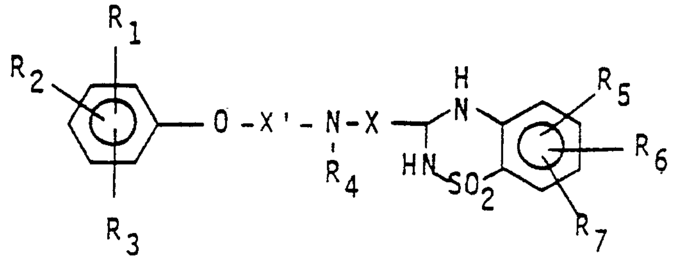

(I)

(die Bedeutung der Symbole ist in der Beschreibung erläutert) können nach an sich bekannten Verfahren

EP 0 264 639 A1

Xerox Copy Centre

hergestellt und als Arzneistoffe verwendet werden.

## Neue Aryloxy-aminoalkane, ihre Herstellung und Verwendung

Die Erfindung betrifft neue 1-Aryloxy-dihydrobenzothiadiazinylalkylamino-alkane, ihre Herstellung nach an sich bekannten Verfahren und die Verwendung der neuen Verbindungen in der Therapie, insbesondere zur Behandlung oder Prophylaxe des Bluthochdrucks.

Die neuen 1-Aryloxy-dihydrobenzothiadiazinylalkylaminoalkane entsprechen der allgemeinen Formel

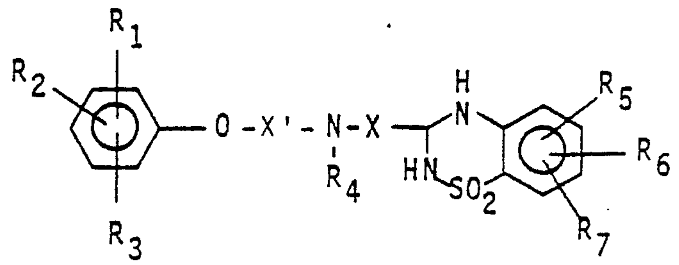

(I),

in der

$R_1$ ein Wasserstoff-oder Halogenatom, eine Niederalkyl-, Niederalkoxy-, Niederalkoxyniederalkyl-, Niederalkenyl-, Niederalkenyloxy-, Cyano-, Cyanoniederalkyl-, Hydroxy-, Trifluormethyl-, Nitro-, Amino-, Acylamino-, Aminocarbonylmethylgruppe,

$R_2$ ein Wasserstoff-oder Halogenatom, eine Niederalkyl-oder niederalkoxygruppe oder eine Acylaminogruppe

$R_3$ ein Wasserstoff-oder Halogenatom, eine Acyl-, eine Niederalkyl-oder Niederalkoxygruppe $R_2$ und $R_3$ gemeinsam auch eine -CH = CH-CH = CH-oder eine Methylendioxygruppe, ferner

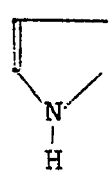

oder

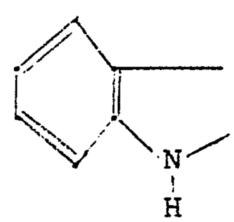

,

$R_4$ ein Wasserstoffatom, eine Niederalkyl-, eine Niederalkenyl-oder eine Niederalkinylgruppe,

$R_5$ ein Chloratom oder eine Trifluormethylgruppe,

$R_6$ ein Chloratom oder eine Sulfonamidgruppe,

$R_7$ ein Wasserstoffatom oder ein Chloratom,

X eine Alkylengruppe und

X' eine Alkylengruppe

bedeutet, und ihre Säureadditionssalze.

Unter "Halogenatome" werden hier Fluor, Chlor und Brom verstanden. Bevorzugt sind Fluor und vor allem Chlor.

Die Niederalkyl-bzw. Niederalkoxygruppen enthalten 1 bis 4, vorzugsweise 1 bis 3 C-Atome; hervorzuheben sind die Gruppen mit 1 bis 2 C-Atomen. die Niederalkenyl-und Niederalkinylgruppen enthalten bis zu 4 C-Atome, wobei in der Regel die Doppel-oder Dreifachbindung sich nicht in 1-Stellung befindet; hervorzuheben sind Allyl und Propargyl. Als "Cyanoniederalkyl" ist vor allem Cyanmethyl zu nennen. Die Alkylengruppe X bzw X' enthält 1 bis 8 C-Atome; bevorzugt weist X bzw X' 1 bis 5 C-Atome, vor allem 1 bis 3 C-Atome auf. Soweit die C-Ketten aus mehreren C-Atomen bestehen, können sie unverzweigt oder verzweigt sein. Dies gilt auch für den Acylrest in der Acylaminogruppe (Definition von $R_1$ und $R_2$). Die Acylgruppe (wie auch die Acylgruppe in der Definition von $R_3$) enthält dabei 1 bis 8, vorzugsweise 2 bis 6 C-Atome und kann aliphatisch oder, bei $C_7$ und $C_8$, aromatisch sein.

Unter einem "aromatischen Rest" wird hier ein Phenyl-oder ein insbesondere durch Halogenatome, Niederalkyl-oder Niederalkoxygruppen substituierter Phenylrest, aber auch ein Benzylrest verstanden.

Enthält die Verbindung der Formel I mehrere Substituenten mit Kohlenstoffketten, so kann die Zahl der Kohlenstoffatome in diesen Ketten gleich oder verschieden sein.

Die Verbindungen der Formel I können als freie Basen oder als Säureadditionssalze vorliegen. Aufgrund ihrer Asymmetriezentren können sie jeweils in Form von Racematen bzw. von Gemischen von Enantiomeren oder in Form der einzelnen Enantiomeren vorliegen.

Geeignete Verfahren zur Herstellung der neuen Verbindungen werden im folgenden aufgeführt.

a) Umsetzung einer Verbindung der Formel

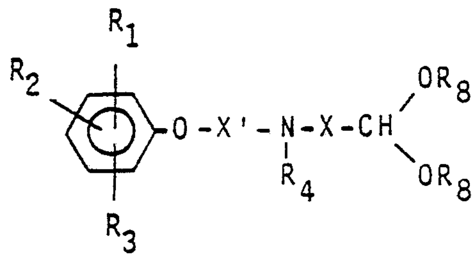

(II),

in der $R_1$, $R_2$, $R_3$, X und X' die obige Bedeutung haben und $R_8$ einen Alkylrest oder Wasserstoff bedeutet, mit einer Verbindung der Formel

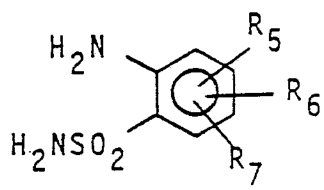

(III),

in der $R_5$ bis $R_7$ die obige Bedeutung haben.

b) Umsetzung einer Verbindung der Formel

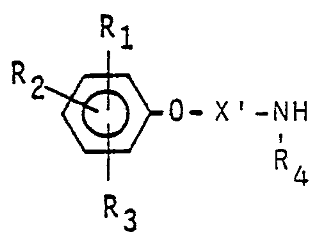

(IV),

in der $R_1$ bis $R_4$ und X'die obige Bedeutung haben, mit einer Verbindung der Formel

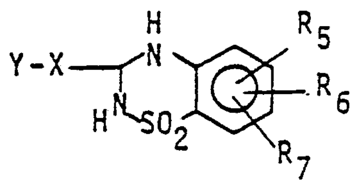

(V),

in der $R_5$ bis $R_7$ und X die obige Bedeutung haben und Y eine Abgangsgruppe wie z.B. Halogen (Cl, Br, J) oder einen Sulfonsäureesterrest bedeutet.

C) Umsetzung einer Verbindung der Formel

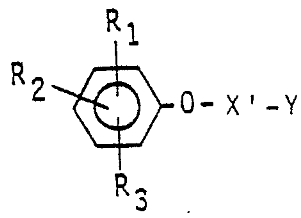

(VI),

in der $R_1$ bis $R_3$, X' und Y die obige Bedeutung haben, mit einer Verbindung der Formel

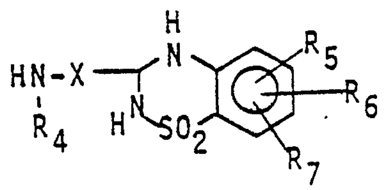

(VII),

in der $R_4$ bis $R_7$ und X die obige Bedeutung haben.

d) Umsetzung einer Verbindung der Formel

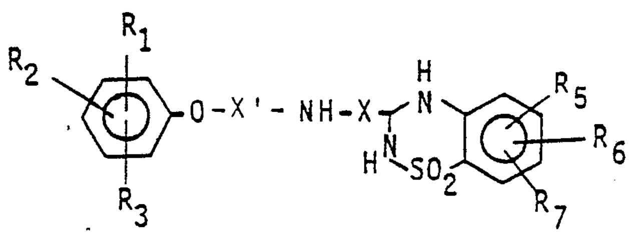

(VIII),

in der $R_1$ bis $R_3$ und $R_5$ bis $R_7$, X und X' die obige Bedeutung haben, mit einer Verbindung umsetzt, die zur Einführung des Restes $R_4$ geeignet ist.

Als solche Verbindungen eignen sich z.B. Verbindungen der Formel

$R_4$-Z (IX),

in der $R_4$ die obige Bedeutung hat und Z eine abspaltbare Gruppe, wie z.B. Cl, Br bedeutet, oder Mittel zur Einführung der Methylgruppe, etwa Dimethylsulfat oder Formaldehyd/Ameisensäure.

e) Umsetzung einer Verbindung der Formel

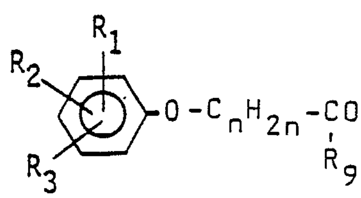

(X)

in der $R_1$ bis $R_3$ die obige Bedeutung haben und $R_9$ einen Niederakylrest und n eine ganze Zahl von 1 bis 6 bedeutet, wobei die Summe der C-Atome in der Gruppe $C_2H_{2n}$-CO-$R_9$ derjenigen in der X'-Gruppe des erhaltenen Endprodukts entspricht, mit einer Verbindung der Formel

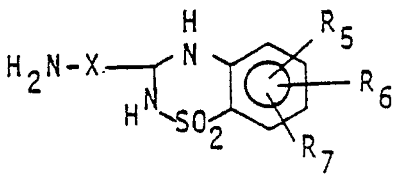

(XI),

in der $R_5$ bis $R_7$ und X die obige Bedeutung haben, unter den Bedingungen der hydrierenden Aminierung.

Soweit nach den Verfahren a) bis e) zunächst Säureadditionssalze erhalten werden, werden diese nach üblichen Methoden gewünschtenfalls in freie Basen oder in Salze anderer Säuren überführt; zunächst erhaltene Basen werden gewünschtenfalls in Säureadditionssalze umgewandelt.

Die Verfahren a) bis d) werden vorzugsweise bei Temperaturen zwischen O und 100°C, insbesondere bei 40 bis 80°C durchgeführt.

Als Reaktionsmedien dienen Alkohole oder andere polare Lösungsmittel, z.B. Methanol, Ethanol, Isopropanol, Dioxan, Tetrahydrofuran, gegebenenfalls in Mischung.

Die Ausgangsverbindungen für die erfindungsgemäßen Verfahren sind bekannt oder können nach bekannten Methoden hergestellt werden.

Die erfindungsgemäßen Verbindungen besitzen mindestens ein asymmetrisches C-Atom und liegen als Racemate oder bei Vorhandensein mehrerer chiraler Zentren als Diastereoisomere vor. Diese können durch Verwendung optisch aktiver Ausgangsverbindungen aufgebaut oder durch Umkristallisation aus geeigneten Lösungsmitteln oder Lösungsmittelgemischen gewonnen werden.

Die erfindungsgemäßen 1-Aryloxy-dihydrobenzothiadiazinylalkylamino-alkane der allgemeinen Formel I können in üblicher Weise in ihre physiologisch verträglichen Säureadditionssalze überführt werden. Geeignete Säuren sind beispielsweise Salzsäure, Bromwasserstoffsäure, Schwefelsäure, Methansulfonsäure, Maleinsäure, Essigsäure, Oxalsäure, Milchsäure oder Weinsäure.

Die Verbindungen der allgemeinen Formel I bzw. deren physiologisch verträgliche Säureadditionssalze haben im Tierversuch wertvolle therapeutische, insbesondere blutdrucksenkende Eigenschaften gezeigt und können daher beispielsweise zur Behandlung oder Prophylaxe des Bluthochdrucks in der Humanmedizin eingesetzt werden. Sie können außerdem als Zwischenprodukte zur Herstellung anderer Arzneistoffe dienen.

Als wertvoll haben sich insbesondere solche Verbindungen der allgemeinen Formel I herausgestellt, bei denen $R_1$ und $R_2$ jeweils ein Chloratom, während $R_3$ Wasserstoff, $R_4$ Wasserstoff, Allyl oder Propargyl, $R_5$ Chlor, $R_6$ Sulfonamido und $R_7$ Wasserstoff oder Chlor ist; oder $R_1$ einen Cyanorest in 2-Position oder einen Methoxyrest in 3-Position oder einen Alkoxyalkylrest in 4-Position bedeutet, in diesen Fällen ist $R_2$ Wasserstoff oder Acylamino (im allgemeinen mit insgesamt 4-6 C-Atomen), während $R_4$ bis $R_7$ die vorstehend genannten Bedeutungen haben. X und X' enthalten jeweils bevorzugt 1-3 C-Atome.

Die einzeldosis der erfindungsgemäßen Substanzen liegt bei 5 bis 300 mg, vorzugsweise bei 20 bis 150 mg (oral).

Die erfindungsgemäßen Wirkstoffe können in die üblichen galenischen Anwendungsformen, wie Tabletten, Dragées, Lösungen, Emulsionen, Pulver, Kapseln oder Depotformen gebracht werden, wobei zu deren Herstellung die üblichen pharmazeutischen Hilfsstoffe sowie die üblichen Fertigungsmethoden herangezogen werden können. Entsprechende Tabletten können beispielsweise durch Mischen der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermittel, wie Magnesiumstearat oder Talk, und/oder Mitteln zur Erzielung eines Depoteffekts, wie Carboxypolymethylen, Carboxymethylcellulose, Celluloseacetatphthalat oder Polyvinylacetat erhalten werden.

Die Tabletten können auch aus mehreren Schichten bestehen. Entsprechend können Dragées durch Überziehen von analog den Tabletten hergestellten Kernen mit üblicherweise in Dragéeüberzügen verwendeten Mitteln, beispielsweise Kollidon oder Schellack, Gummi arabicum, Talk,. Titandioxid oder Zucker, hergestellt werden. Zur Erzielung eines Depoteffekts oder zur Vermeidung von Inkompatibilitäten kann der Kern auch aus mehreren Schichten bestehen. Desgleichen kann auch die Dragéehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe bzw. Wirkstoffkombinationen können zusätzlich noch ein Süßungsmittel, wie Saccharin, Cyclamat, Glycerin oder Zucker, sowie ein geschmackverbesserndes Mittel, z.B. Aromastoffe, wie Vanillin oder Orangenextrakt, enthalten.

Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Komplexonen, hergestellt und in Injektionsflaschen oder Ampullen abgefüllt.

Die Wirkstoffe bzw. Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen der dafür vorgesehenen Wirkstoffe bzw. Wirkstoffkombinationen mit üblichen Trägermitteln, wie Neutralfetten oder Polyäthylenglykol bzw. dessen Derivaten, herstellen.

Die erfindungsgemäßen Verbindungen sind auch für die Kombination mit anderen pharmakodynamisch wirksamen Stoffen wie z.B. β-Adrenolytika, Diuretika, Calciumantagonisten oder Tranquilizern geeignet.

Formulierungsbeispiele

1. Tabletten

| | |
|---|---|
| Verbindung nach Beispiel 9 | 40,5 mg |
| Maisstärke | 164,0 mg |
| sek. Calciumphosphat | 240,0 mg |
| Magnesiumstearat | 1,0 mg |
| | 445,0 mg |

Herstellung:

Die einzelnen Bestandteile werden intensiv miteinander vermischt und die Mischung in üblicher Weise granuliert. Das Granulat wird zu Tabletten von 445 mg Gewicht verpreßt, von denen jede 40 mg Wirkstoff enthält.

Anstelle des in diesem Beispiel genannten Wirkstoffs können andere erfindungsgemäße Verbindungen in gleicher oder in abweichender Menge verwendet werden.

2. Gelatine Kapseln

Der Inhalt der Kapsel setzt sich wie folgt zusammen:

| | |
|---|---|
| Verbindung nach Beispiel 13 | 25,0 mg |
| Maisstärke | 175,0 mg |
| | 200,0 mg |

Herstellung:

Die Bestandteile des Kapselinhalts werden intensiv vermischt und 200 mg-Portionen der Mischung werden in Gelatine-Kapseln geeigneter Größe abgefüllt. Jede Kapsel enthält 25 mg des Wirkstoffs.

An genetisch hypertonen Ratten wurde die Wirkung der erfindungsgemäßen Verbindungen auf den Blutdruck untersucht; die Dosis betrug jeweils 30 mg/kg p.o.

| Beispiel | Blutdrucksenkung in mm Hg/mbar |
|---|---|
| 7 | -63 / -84 |
| 9 | -60 / -80 |
| 13 | -44 / -59 |
| 18 | -56 / -75 |
| 22 | -55 / -73 |
| 26 | -47 / -63 |

Einige Befunde sprechen dafür, daß die blutdrucksenkende Wirkung der erfindungsgemäßen Verbindungen auf einem neuen Wirkungsmechanismus beruht. Ein wesentlicher Vorteil der neuen Verbindungen ist daher, daß dem Therapeuten eine neue Behandlungsmöglichkeit geboten wird, falls die üblichen Blutdrucksenker nicht verwendbar sind.

Bei den neuen Verbindungen zeigt sich ein relativ langsamer Wirkungseintritt; vorteilhaft ist die geringe Toxizität. Auch zeigt sich kein Einfluß auf die Herzfrequenz und keine Bradycardie.

Herstellungsbeispiele

Beispiel 1

1-(2-Cyanophenoxy)-3-[(6-chlor-7-sulfonamido-1,1-dioxo-dihydrobenzothiadiazinyl)-3-methylamino]-propan-hydrochlorid

9,7 g (0,028 Mol) N-3-(2-Cyanophenoxy)-1-propylaminoacetaldehyd-dimethylacetal-hydrobromid werden zusammen mit 8 g 3-Chloranilino-4,6-disulfonamid in 70 ml Ethanol nach Zugabe von 3 g p-Toluolsulfonsäure sechs Stunden unter Rühren zum Sieden erhitzt. Nach eintägigem Stehen bei Raumtemperatur wird von ungelösten Anteilen abgetrennt, das Filtrat i.V. eingeengt und der Rückstand über eine Kieselgelsäule gereinigt. Laufmittelmischung: 700 Teile Essigester, 300 Teile Isopropanol, 10 Teile Ammoniak. Nach Vereinigen der einheitlichen Fraktion und Abdestillieren des Lösungsmittelgemischs wird der verbleibende Rückstand in 40 ml Aceton gelöst und filtriert. Das Filtrat wird mit alkaoholischer HCl angesäuert und mit etwas Ether versetzt. Das auskristallisierte Hydrochlorid wird abgesaugt und mit Aceton und Ether nachgewaschen. Nach Trockung werden 2,6 g farbloses Kristallisat gewonnen;
Fp: 145-147°C..

Beispiel 2

1-(2,4-Dichlorphenoxy)-3-[(6-chlor-7-sulfonamido-1,1-dioxo-dihydrobenzothiadiazinyl)-3-methylamino]-propan-hydrochlorid

8,3 g (0,021 Mol) N-3-(2,4-Dichlorphenoxy)-1-propylaminoacetaldehyddimethylacetalhydrochlorid werden in 70 ml Ethanol unter Rühren suspendiert und nach Zugabe von 6,1 g (0,021 Mol) 3-Chloranilino-4,6-disulfonamid und 3 g p-Toluolsulfonsäure acht Stunden zum Sieden unter Rückfluß erhitzt. Nach 3-tägigem Stehen bei Raumtemperatur wird von ungelösten Anteilen abgetrennt, das Filtrat i.V. eingeengt und der Rückstand über eine Kieselgelsäule gereinigt. Laufmittelgemisch: 700 Teile Essigester, 300 Teile Isopropanol, 10 Teile Ammoniak. Aus den im DC einheitlichen Fraktionen wurden nach Abdestillieren des Lösungsmittelgemischs 1,7 g Reinsubstanz isoliert. Die in Aceton gelöste Verbindung ließ sich nach Zugabe von alkoholischer HCl als farbloses Hydrochlorid gewinnen (1,2 g);
Fp: 153-155°C.

In Analogie zu Beispiel 2 wird auch das Beispiel 3 synthetisiert:

Beispiel 3

1-(3-Methoxyphenoxy)-3-[(6-chlor-7-sulfonamido-1,1-dioxo-dihydrobenzothiadiazinyl)-3-methylamino]-propan-hydrochloridFp: 116-118°C.

Beispiel 4

1-(3-Methoxyphenoxy)-N-methyl-3-[(6-chlor-7-sulfonamido-1,1-dioxo-dihydrobenzothiadiazinyl)-3-methylamino]-propan-oxalat

5,7 g (0,02 Mol) N-Methyl-N-3-(3-methoxy-phenoxy)-propylaminoacetaldehyddimethylacetal werden mit 5,8 g (0,02 Mol) 3-Chloranilino-4,6-disulfonamid in 70 ml Dioxan gelöst und mit etherischer HCl angesäuert. Nach Abdestillieren des Dioxans wird der Rückstand nach Zugabe von 2,5 g p-Toluolsulfonsäure und 80 ml Dioxan 45 Minuten unter Rühren zum Sieden erhitzt, wobei sich ölige Anteile abscheiden. Das Lösungsmittel wird i.V. abdestilliert und der Rückstand über eine Kieselgelsäule gereinigt. Lösungsmittelgemisch: 700 Teile Essigester, 300 Teile Isopropanol, 50 Teile Ammoniak. Nach Eindampfen des Eluats wird der Rückstand in Aceton gelöst und in eine Lösung von 5 g Oxalsäure in Aceton getropft. Nach Zugabe von Ether kristallisiert das Oxalat farblos aus. Das Kristsllisat wird aus Acetonitril umkristallisiert, wonach 2,1 g Oxalat gewonnen werden;
Fp: 120-123°C.

In Analogie zu Beispiel 4 werden die Verbindungen der Beispiele 5 und 6 erhalten.

Beispiel 5

1-(2,4-Dichlorphenoxy)-N-methyl-3-[(6-chlor-7-sulfonamido-1,1-dioxo-dihydrobenzothiadiazinyl)-3-methylamino]-propanhydrochlorid Fp: 165-167°C.

Beispiel 6

1-(2-Cyanophenoxy)-N-methyl-3-[(6-chlor-7-sulfonamido-1,1-dioxo-dihydrobenzothiadiazinyl)-3-methylamino]-propan-hydrochlorid Fp: 153-156°C.

Beispiel 7

1-(2,6-dimethylphenoxy)-2-[(6-chlor-7-sulfonamido-1,1-dioxo-dihydrobenzothiadiazinyl)-3-methylamino]-ethanhydrochlorid

8,6 g (0,034 Mol) N-2-(2,6-Dimethylphenoxy)-1-ethylaminoacetaldehyddimethylacetal werden in 100 ml Dioxan gelöst, 9,7 g (0,034 Mol) 3-Chloranilino-4,6-disulfonamid zugegeben und etherische HCl eingetropft. Das Lösungsmittel wird i.V. abdestilliert, der Rückstand in 100 ml Dioxan aufgenommen und 4 g p-Toluolsulfonsäure zugegeben. Nach 3-stündigem Sieden unter Rückfluß wird das Dioxan i.V. abdestilliert und der Rückstand über eine Kieselgelsäule gereinigt. Lösungsmittelgemisch: 700 Teile Essigester, 300 Teile Isopropanol. Nach Einengen des Eluats wird der Rückstand in Aceton gelöst, mit etherischer HCl angesäuert und weiter Ether zugegeben, bis das Hydrochlorid langsam auskristallisiert. Es werden 2,9 g farbloses Kristallisat gewonnen;
Fp. 156-158°C.

Beispiel 8

1-[4-(2-Methoxyethyl)-phenoxy)-2-[(6-chlor-7-sulfonamido-1,1-dioxo-dihydrobenzothiadiazinyl)-3-methylamino]-ethanhydrochlorid

10,3 g (0,037 Mol) N-2-[4-(2-Methoxyethyl)-phenoxy]-1-ethylaminoacetaldehyddimethylacetal werden zusammen mit 10,4 g (0,037 Mol) 3-Chloranilino-4,6-disulfonamid in 100 ml Methanol gelöst und etherische HCl zugegeben. Nach Abdestillieren der Lösungsmittel wird der Rückstand mit 100 ml Dioxan digeriert und 4 g p-Toluolsulfonsäure zugegeben. Unter Rühren wird fünf Stunden am Rückfluß zum Sieden erhitzt. Nach Erkalten werden 8,2 g Kristallisat isoliert. Es wird aus Methanol unter Zugabe von Ether umkristallisiert, wonach 5,7 g Hydrochlorid erhalten werden;
Fp: 197-198°C.

Beispiel 9

1-(3-Methoxyphenoxy)-2-[(6-chlor-7-sulfonamido-1,1-dioxo-dihydrobenzothiadiazinyl)-3-methylamino]-ethanhydrochlorid

8 g (0,031 Mol) N-2-(3-Methoxyphenoxy)-1-ethylaminoacetaldehyddimethylacetal werden in 75 ml Ethanol gelöst, 9 g (0,031 Mol) 3-Chloranilino-4,6-disulfonamid zugegeben und nach Zusatz von 4 g p-Toluolsulfonsäure mit alkoholischer HCl angesäuert. Nach 15-stündigem Sieden unter Rückfluß wird das Lösungsmittel i.V. abdestilliert und der Rückstand über eine Kieselgelsäule gereinigt. Der aus dem Eluat gewonnene Rückstand wird in Aceton gelöst und mit alkoholischer HCl unter Zugabe von Ether zum Kristallisieren gebracht. Man erhält 3,8 g farbloses Hydrochlorid;
Fp. 187-189°C.

In Analogie zu Beispiel 7 bis 9 werden nach folgende Substanzen erhalten:

Beispiel 10

1-(3-Methylphenoxy)-2-[(6-chlor-7-sulfonamido-1,1-dioxo-dihydrobenzothiadiazinyl)-3-methylamino]-ethanhydrochlorid Fp. 195-197°C.

Beispiel 11

1-α-Naphtoxy)-2-[(6-chlor-7-sulfonamido-1,1-dioxo-dihydrobenzothiadiazinyl)-3-methylamino]-ethanhydrochlorid Fp: 169-172°C.

Beispiel 12

1-(4-Methylphenoxy)-2-[(6-chlor-7-sulfonamido-1,1-dioxo-dihydrobenzothiadiazinyl)-3-methylamino]-ethanhydrochlorid Fp: 201-203°C.

Beispiel 13

1-(2-Cyanophenoxy)-2-[(6-chlor-7-sulfonamido-1,1-dioxo-dihydrobenzothiadiazinyl)-3-methylamino]-ethanhydrochlorid Fp: 210-212°C.

Beispiel 14

1-(2,4-Dichlorphenoxy)-2-[(6-chlor-7-sulfonamido-1,1-dioxo-dihydrobenzothiadiazinyl)-3-methylamino]-ethanhydrochlorid Fp: 210-212°C.

Beispiel 15

1-(3,4-Dmethoxyphenoxy)-2-[(6-chlor-7-sulfonamido-1,1-dioxo-dihydrobenzothiadiazinyl)-3-methylamino]-ethan-hydrochlorid Fp: 197-199°C.

Beispiel 16

1-(3-Methoxyphenoxy)-N-methyl-2-[(6-chlor-7-sulfonamido-1,1-dioxo-dihydrobenzothiadiazinyl)-3-methylamino]-ethan-hydrochlorid

10,5 g (0,039 Mol) N-Methyl-N-2-(3-methoxy-phenoxy)-ethylaminoacetaldehyddimethylacetal werden in 100 ml Ethanol gelöst, 4 g p-Toluolsulfonsäure und 11,2 g (0,039 Mol) 3-Chloranilino-4,6-disulfonamid zugegeben und alkoholische HCl zugetropft. Unter Rühren wird 12 Stunden zum Sieden am Rückfluß erhitzt. Nach Erkalten wird i.V. auf ca. 50 ml eingeengt und mit Ammoniak alkalisch gestellt, wobei sich ein Niederschlag bildet. Nach Filtration wird das Filtrat zur Trockne eingedampft und der Rückstand über eine Kieselgelsäule gereinigt. Lösungsmittelgemisch: 700 Teile Essigester, 300 Teile Isopropanol. Aus dem Eluat werden 6,1 g Rückstand gewonnen, der in Aceton gelöst, in eine Lösung von Oxalsäure in Aceton eingerührt wird. Nach Zugabe von Äther kristallisiert das Oxalat (2,7 g) aus;
Fp. 168-170°C.

Beispiel 17

1-(3-Methoxyphenoxy)-N-ethyl-2-[(6-chlor-7-sulfonamido-1,1-dioxo-dihydrobenzothiadiazinyl)-3-methylamino]-ethan-hydrochlorid

2,05 g (0,004 Mol) 1-(3-Methoxyphenoxy)-2-[(6-chlor-7-sulfonamido-1,1-dioxo-dihydrobenzothiadiazinyl)-methylamino]-ethanhydrochlorid werden in 80 ml Methanol mit 4 ml 1N NaOH vereinigt. Das Lösungsmittel wird i.V. abdestilliert und der rückstand mit einem Gemisch aus 1,5 g (0,0054 Mol) Ethyljodid, 400 mg Natriumbikarbonat, 8 ml Dimethylformamid und 20 ml Tetrahydrofuran versetzt. Das Ganze wird zwei Stunden unter Rückfluß zum Sieden erhitzt. Nach Abdestillieren der Lösungsmittel wird der rückstand über eine Kieselgelsäule gereinigt. Lösungsmittelgemisch: 700 Teile Essigester, 300 Teile Isopropanol, 10 Teile Ammoniak. Der aus dem Eluat gewonnene Rückstand wird in Aceton gelöst, etherische HCl zugegeben und durch weitere Zugabe von Ether das Hydrochlorid abgeschieden. Es werden 0,9 g amorphe Substanz erhalten, die beim Erhitzen sintert und dann Zersetzungserscheinungen zeigt.

In Analogie zu Beispiel 17 wird Beispiel 18 synthetisiert:

Beispiel 18

1-(3-Methoxyphenoxy)-N-allyl-2-[(6-chlor-7-sulfonamido-1,1-dioxo-dihydrobenzothiadiazinyl)-3-methylamino]-ethan-hydrochlorid Fp: Zersetzung

Beispiel 19

1-(3-Methoxyphenoxy)-2-[(6-chlor-7-sulfonamido-1,1-dioxo-dihydrobenzothiadiazinyl)-3-methylamino]-ethan-hydrochlorid

3,5 g (0,012 Mol) N-2-(3-Methoxyphenoxy)-1-ethyl-3-aminopropionaldehyddiethylacetal und 3,4 g (0,012 Mol) 3-Chloranilino-4,6-disulfonamid werden in 60 ml Dioxan gelöst, etherische HCl zugegeben und nach abdestillieren des Lösungsmittels 60 ml Dioxan und 2,3 g p-Toluolsulfonsäure zugegeben. Nach zweistündigem Sieden wird das Lösungsmittel abdestilliert und der Rückstand über eine Kieselgelsäule wie in Beispiel 17 gereinigt. Die isolierte Base wird in Aceton mit alkoholischer HCl angesäuert und durch Zugabe von Ether das Hydrochlorid nach längerem Stehen erhalten;
Fp. 167-169°C.

In Analogie zu Beispiel 19 werden die Verbindungen 20 und 21 erhalten.

Beispiel 20

1-(4-Methoxyphenoxy)-2-[(6-chlor-7-sulfonamido-1,1-dioxo-dihydrobenzothiadiazinyl)-3-ethylamino]-ethan-hydrochlorid Fp: 155-160°C.

Beispiel 21

1-(3-Methoxyphenoxy)-N-methyl-2-[(6-chlor-7-sulfonamido-1,1-dioxo-dihydrobenzothiadiazinyl)-3-ethylamino]-ethan-hydrochlorid Fp: Zersetzung

Beispiel 22

1-(3-Methoxyphenoxy)-2-[(6-trifluormethyl-7-sulfonamido-1,1-dioxo-dihydrobenzothiadiazinyl)-3-ethylamino]-ethan-hydrochlorid

1,6 g (0,006 Mol) N-2-(3-Methoxyphenoxy)--1-ethyl-2-aminoacetaldehyddimethylacetal werden in 400 ml Dioxan gelöst und nach Zugabe von 2 g (0,006 Mol) 3-Chloranilino-4,6-disulfonamid mit alkoholischer HCl versetzt. Nach einstündigem Sieden am Rückfluß wird mit Ammoniak alkalisch gestellt, i.V. eingeengt und der Rückstand wie in Beispiel 17 über eine Kieselgelsäule gereinigt. Die Base wird in Aceton gelöst, etherische HCl zugegeben und durch Zusatz von Ether das Hydrochlorid als farbloses Kristallisat gewonnen (1,3 g);
Fp. 187-190°C.

In Analogie zu Beispiel 22 wird die folgende Verbindung erhalten:

Beispiel 23

1-(3-Methoxyphenoxy)-2-[(6-trifluormethyl-7-sulfonamido-1,1-dioxo-dihydrobenzothiadiazinyl)-3-methylamino]-ethan-hydrochlorid Fp: amorphe Substanz, Zersetzung

Beispiel 24

1-(3-Methoxyphenoxy)-2-[(6,7-dichlor-1,1-dioxo-dihydrobenzothiadiazinyl)-3-methylamino]-ethan-hydrochlorid

4,82 g (0,02 Mol) 3,4-Dichloranilino-6-sulfonamid werden in 75 ml Dioxan eingerührt, 5,1 g (0,02 Mol) N-2-(3-Methoxyphenoxy)-1-ethyl-2-aminoacetaldehyddimethyl-acetal eingetragen und etherische HCl zugetropft. Nach Abdestillieren der Lösungsmittel werden 75 ml Dioxan und 2 g p-Toluolsulfonsäure zugegeben. Eine Stunde wird unter Rückfluß zum Sieden erhitzt. Die sich abscheidende Substanz wird isoliert, in Methanol digeriert, Ammoniak zugegeben, filtriert und durch Zugabe von etherischer HCl das Hydrochlorid zur abscheidung gebracht. Es werden 2,9 g Kristallisat gewonnen;
Fp: 182-183°C.

In analoger Weise werden die Verbindungen der nachfolgenden Beispiele erhalten:

Beispiel 25

1-(4-Methoxyethylphenoxy)-2-[(6,7-dichlor-1,1-dioxo-dihydrobenzothiadiazinyl)-3-methylamino]-ethan-hydrochlorid Fp: 187-189°C.

Beispiel 26

1-(4-Methoxyethylphenoxy)-2-[(6,8-dichlor-1,1-dioxo-dihydrobenzothiadiazinyl)-3-methylamino]-ethan-hydrochlorid Fp: 195-197°C.

Beispiel 27

1-(3-Methoxyphenoxy)-2-[(6,8-dichlor-1,1-dioxo-dihydrobenzothiadiazinyl)-3-methylamino]-ethan-hydrochlorid Fp: 200-201 °C.

Beispiel 28

1-(3-Methoxyphenoxy)-2-[(5,6-dichlor-7-sulfonamido-1,1-dioxo-dihydrobenzothiadiazinyl)-3-methylamino]-ethan-hydrochlorid Fp: 190-192 °C.

Beispiel 29

1-(4-Methoxyphenoxy)-2-[(5,6-dichlor-7-sulfonamido-1,1-dioxo-dihydrobenzothiadiazinyl)-3-methylamino]-ethan-hydrochlorid Fp: 185-188 °C.

Beispiel 30

1-(2-Allylphenoxy)-2-[(6-chlor-7-sulfonamido-1,1-dioxo-dihydrobenzothiadiazinyl)-3-methylamino]-ethan-hydrochlorid Fp: 144-147 °C.

Beispiel 31

1-(3-Nitrophenoxy)-2-[(6-chlor-7-sulfonamido-1,1-dioxo-dihydrobenzothiadiazinyl)-3-methylamino]-ethan-hydrochlorid Fp: 184-186 °C.

Beispiel 32

1-(2,6-Dimethylphenoxy)-2-[(6-chlor-7-sulfonamido-1,1-dioxo-dihydrobenzothiadiazinyl)-3-methylamino]-ethan-hydrochlorid Fp: 192-194 °C.

Beispiel 33

1-(2-Allyloxyphenoxy)-2-[(6-chlor-7-sulfonamido-1,1-dioxo-dihydrobenzothiadiazinyl)-3-methylamino]-propan-hydrochlorid Fp: 152-154 °C.

Beispiel 34

1-(3-Methoxyphenoxy)-2-[(6-chlor-7-sulfonamido-1,1-dioxo-dihydrobenzothiadiazinyl)-3-methylamino]-propan-hydrochlorid Fp: 189-192 °C.

Beispiel 35

1-(2-Cyanophenoxy)-2-[(6-chlor-7-sulfonamido-1,1-dioxo-dihydrobenzothiadiazinyl)-3-methylamino]-propan-hydrochlorid Fp: 193-194 °C.

Beispiel 36

1-(2-Cyano-4-isobutyroylamidophenoxy)-2-[(6-chlor-7-sulfonamido-1,1-dioxo-dihydrobenzothiadiazinyl)-3-methylamino]-ethan-hydrochlorid Fp: 140-142°C.

Beispiel 37

1-(2-Bromphenoxy)-2-[(6-chlor-7-sulfonamido-1,1-dioxo-dihydrobenzothiadiazinyl)-3-methylamino]-ethan-hydrochlorid Fp: 155-158°C.

Beispiel 38

1-(3,4-Methylendioxyphenoxy)-2-[(6-chlor-7-sulfonamido-1,1-dioxo-dihydrobenzothiadiazinyl)-3-methylamino]-ethan-hydrochlorid Fp: 211-213°C.

Beispiel 39

1-(2,4,5-Trichlorphenoxy)-2-[(6-chlor-7-sulfonamido-1,1-dioxo-dihydrobenzothiadiazinyl)-3-methylamino]-ethan-hydrochlorid Fp: 174-177°C.

Beispiel 40

1-(2-Allylphenoxy)-N-methyl-2-[(6-chlor-7-sulfonamido-1,1-dioxo-dihydrobenzothiadiazinyl)-3-methylamino]-ethan-hydrochlorid Fp: 156-159°C.

Beispiel 41

1-(2-Allyloxyphenoxy)-N-methyl-2-[(6-chlor-7-sulfonamido-1,1-dioxo-dihydrobenzothiadiazinyl)-3-methylamino]-ethan-hydrochlorid Fp: 164-165°C.

Beispiel 42

1-(2-Bromphenoxy)-N-methyl-2-[(6-chlor-7-sulfonamido-1,1-dioxo-dihydrobenzothiadiazinyl)-3-methylamino]-ethan-hydrochlorid Fp: 168-169°C.

Beispiel 43

1-(3-Nitrophenoxy)-N-methyl-2-[(6-chlor-7-sulfonamido-1,1-dioxo-dihydrobenzothiadiazinyl)-3-methylamino]-ethan-hydrochlorid Fp: 169-171°C.

Beispiel 44

1-(4-Hydroxyphenoxy)-2-[(6-chlor-7-sulfonamido-1,1-dioxo-dihydrobenzothiadiazinyl)-3-methylamino]-ethan-hydrochlorid Fp: 179-181°C.

Beispiel 45

1-(3-Bromphenoxy)-2-[(6-chlor-7-sulfonamido-1,4-dioxo-dihydrobenzothiadiazinyl)-3-methylamino]-ethan-hydrochlorid Fp: 182-184°C.

Beispiel 46

1-(2-Methoxyphenoxy)-2-[(6-chlor-7-sulfonamido-1,1-dioxo-dihydrobenzothiadiazinyl)-3-methylamino]-ethan-hydrochlorid Fp: 220-223°C.

Beispiel 47

1-(2-Cyanomethyl-5-methoxyphenoxy)-2-[(6-chlor-7-sulfonamido-1,1-dioxo-dihydrobenzothiadiazinyl)-3-methylamino]-ethan-hydrochlorid Fp: 184-186°C.

Beispiel 48

1-(2-Methoxyphenoxy)-N-n-propyl-2-[(6-chlor-7-sulfonamido-1,1-dioxo-dihydrobenzothiadiazinyl)-3-methylamino]-ethan-hydrochlorid Fp: 145-148°C.

Beispiel 49

1-(3-Ethoxyphenoxy)-2-[(6-chlor-7-sulfonamido-1,1-dioxo-dihydrobenzothiadiazinyl)-3-methylamino]-ethan-hydrochlorid Fp: 177-180°C.

Beispiel 50

1-(Phenoxy)-2-[(6-chlor-7-sulfonamido-1,1-dioxo-dihydrobenzothiadiazinyl)-3-methylamino]-ethan-hydrochlorid Fp: 193-195°C.

Beispiel 51

1-(4-Ethoxyphenoxy)-2-[(6-chlor-7-sulfonamido-1,1-dioxo-dihydrobenzothiadiazinyl)-3-methylamino]-ethan-hydrochlorid Fp: 193-196°C.

Beispiel 52

1-(3-Methoxyphenoxy)-N-propargyl-2-[(6-chlor-7-sulfonamido-1,1-dioxo-dihydrobenzothiadiazinyl)-3-methylamino]-ethan-hydrochlorid Fp: 160-162°C.

Beispiel 53

1-(2,4-Dichlorphenoxy)-N-propargyl-2-[(6-chlor-7-sulfonamido-1,1-dioxo-dihydrobenzothiadiazinyl)-3-methylamino]-ethan-hydrochlorid Fp: 153-155°C.

Beispiel 54

1-(α-Naphthoxy)-N-propargyl-2-[(6-chlor-7-sulfonamido-1,1-dioxo-dihydrobenzothiadiazinyl)-3-methylamino]-ethan-hydrochlorid Fp: 86-88°C.

Beispiel 55

1-(2,4-Dichlorphenoxy)-2-[(6-dichlor-1,1-dioxo-dihydrobenzothiadiazinyl)-3-methylamino]-propan-hydrochlorid Fp: 190-192°C.

Beispiel 56

1-(2,4-Dichlorphenoxy)-2-[(5,6-dichlor-7-sulfonamido-1,1-dioxo-dihydrobenzothiadiazinyl)-3-methylamino]-propan-hydrochlorid Fp: 170-172°C.

Beispiel 57

1-(4-Methoxyethylphenoxy)-N-propargyl-2-[(6-chlor-7-sulfonamido-1,1-dioxo-dihydrobenzothiadiazinyl)-3-methylamino]-ethan-hydrochlorid Fp: 168-171°C.

Nach den erfindungsgemäßen Verfahren können auch die folgenden Verbindungen hergestellt werden:

1-(4-Carbazolyloxy)-2-[(6-chlor-7-sulfonamido-1,1-dioxo-dihydrobenzothiadiazinyl)-3-methylamino]-ethan-hydrochlorid

1-(4-Indolyloxy)-2-[(6-chlor-7-sulfonamido-1,1-dioxo-dihydrobenzothiadiazinyl)-3-methylamino]-ethan-hydrochlorid

1-(2-Acetyl-4-butyroylamidophenoxy)-2-[(6-chlor-7-sulfonamido-1,1-dioxo-dihydrobenzothiadiazinyl)-3-methylamino]-ethan-hydrochlorid

1-(3-Hydroxyphenoxy)-2-[(6-chlor-7-sulfonamido-1,1-dioxo-dihydrobenzothiadiazinyl)-3-methylamino]-ethan-hydrochlorid

1-(2-Allylphenoxy)-2-[(6-chlor-7-sulfonamido-1,1-dioxo-dihydrobenzothiadiazinyl)-3-methylamino]-propan-hydrochlorid

1-(2-Allyloxyphenoxy)-2-[(6-chlor-7-sulfonamido-1,1-dioxo-dihydrobenzothiadiazinyl)-3-methylamino]-propan-hydrochlorid

1-(2-Bromphenoxy)-2-[(6-chlor-7-sulfonamido-1,1-dioxo-dihydrobenzothiadiazinyl)-3-methylamino]-propan-hydrochlorid

1-(3-Nitrophenoxy)-2-[(6-chlor-7-sulfonamido-1,1-dioxo-dihydrobenzothiadiazinyl)-3-methylamino]-propan-hydrochlorid

## Ansprüche

1. Verbindungen der Formel

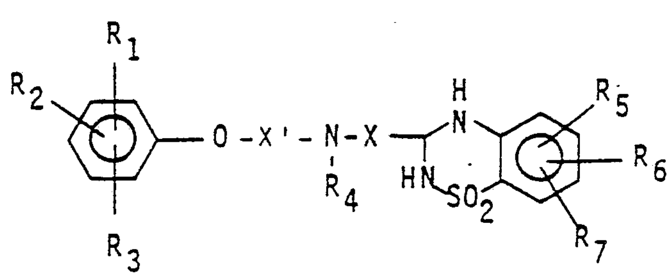

(I),

in der

$R_1$ ein Wasserstoff-oder Halogenatom, eine Niederalkyl-, Niederalkoxy-, Niederalkoxyniederalkyl-, Niederalkenyl-, Niederalkenyloxy-, Cyano-, Cyanoniederalkyl-, Hydroxy-, Trifluormethyl-, Nitro-, Amino-, Acylamino-, Aminocarbonylmethylgruppe,

$R_2$ ein Wasserstoff-oder Halogenatom, eine Niederalkyl-oder Niederalkoxygruppe oder eine Acylaminogruppe

$R_3$ ein Wasserstoff-oder Halogenatom, eine Acyl-, eine Niederalkyl-oder Niederalkoxygruppe $R_2$ und $R_3$ gemeinsam auch eine -CH = CH-CH = CH-oder eine Methylendioxygruppe, ferner

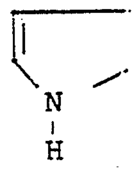

oder -

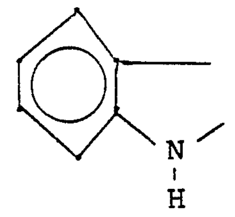

,

$R_4$ ein Wasserstoffatom, eine Niederalkyl-, eine Niederalkenyl-oder eine Niederalkinylgruppe,

$R_5$ ein Chloratom oder eine Trifluormethylgruppe

$R_6$ ein Chloratom oder eine Sulfonamidgruppe

$R_7$ ein Wasserstoffatom oder ein Chloratom

X eine Alkylengruppe und

X' eine Alkylengruppe

bedeutet, und ihre Säureadditionssalze.

2. Verbindungen der Formel I, worin

$R_1$ Chlor, Brom, CN, $NO_2$, OH, $CH_2CN$, Methoxy, Ethoxy, Methyl, Ethyl, Allyl, Allyloxy, Methoxyethyl oder Wasserstoff,

$R_2$ Wasserstoff, Chlor, Methyl, Methoxy, Niederacylamino,

$R_3$ Wasserstoff, Chlor, Niederacyl, zusammen mit $R_2$ auch -CH = CH-CH = CH, O-$CH_2$-O, CH = CH-NH-oder

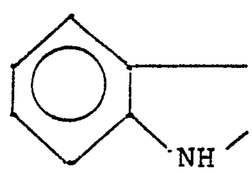

,

$R_4$ Wasserstoff, $C_1$-$C_3$-Alkyl, Allyl, Propargyl,

$R_5$ Chlor, $CF_3$,

$R_6$ Chlor, $SO_2NH_2$,

$R_7$ Wasserstoff

X $(CH_2)_n$ (mit n = 1 oder 2)

X' $(CH_2)_m$ (mit m = 1, 2 oder 3)

bedeutet.

3. 1-(3-Methoxyphenoxy)-2-[(6-chlor-7-sulfonamido-1,1-dioxo-dihydrobenzothiadiazinyl)-3-methylamino]-ethan und seine Säureadditionssalze.

4. 1-(2-Cyanophenoxy)-2-[(6-chlor-7-sulfonamido-1,1-dioxo-dihydrobenzothiadiazinyl)-3-methylamino]-ethan und seine Säureadditionssalze.

5. 1-(3-Methoxyphenoxy)-2-[(6-trifluormethyl-7-sulfonamido-1,1-dioxo-dihydrobenzothiadiazinyl)-3-ethylamino]-ethan und seine Säureadditionssalze.

6. 1-(2,6-Dimethylphenoxy)-2-[(6-chlor-7-sulfonamido-1,1-dioxo-dihydrobenzothiadiazinyl)-3-methylamino]-ethan und seine Säureadditionssalze.

7. 1-(3-Methoxyphenoxy)-N-allyl-2-[(6-chlor-7-sulfonamido-1,1-dioxo-dihydrobenzothiadiazinyl)-3-methylamino]-ethan und seine Säureadditionssalze.

8. 1-(4-Methoxyethylphenoxy)-2-[(6,8-dichlor-1,1-dioxo-dihydrobenzothiadiazinyl)-3-methylamino]-ethan und seine Säureadditionssalze.

9. Mittel zur Behandlung oder Prophylaxe des Bluthochdrucks, gekennzeichnet durch einen Gehalt an einer Verbindung nach Anspruch 1 bis 8 neben üblichen Hilfs-und/oder Trägerstoffen.

10. Verwendung von Verbindungen nach Anspruch 1 bis 8 bei der Behandlung oder Prophylaxe des Bluthochdrucks.

11. Verfahren zur Herstellung von Verbindungen gemäß Anspruch 1 bis 8 nach an sich bekannten Methoden, dadurch gekennzeichnet, daß man

a) eine Verbindung der Formel

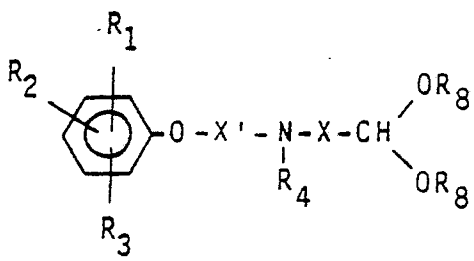

(II),

mit einer Verbindung der Formel

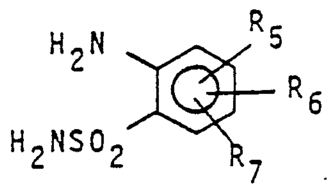

(III),

umsetzt oder daß man

b) eine Verbindung der Formel

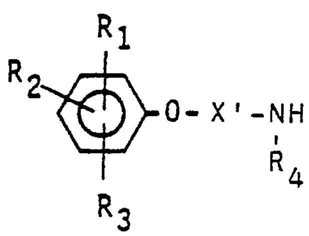

(IV)

mit einer Verbindung der Formel

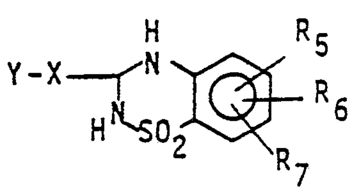

(V)

umsetzt oder daß man

c) eine Verbindung der Formel

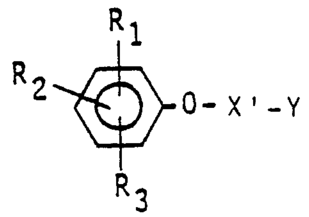

(VI)

mit einer Verbindung der Formel

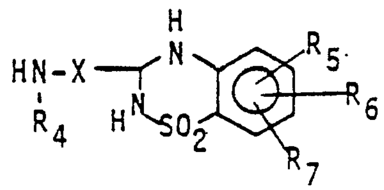

(VII)

umsetzt oder daß man

d) in eine Verbindung der Formel

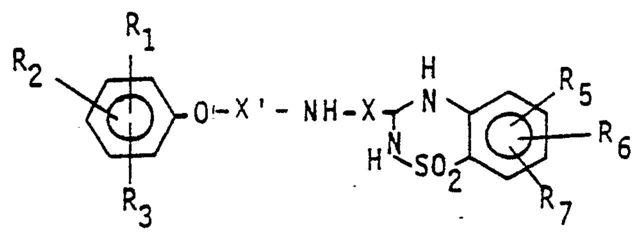

(VIII)

mit üblichen Mitteln den Rest $R_4$ einführt oder daß man

e) eine Verbindung der Formel

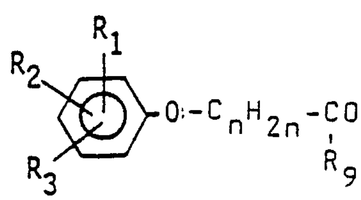

(X)

einer hydrierenden Aminierung mit einer Verbindung der Formel

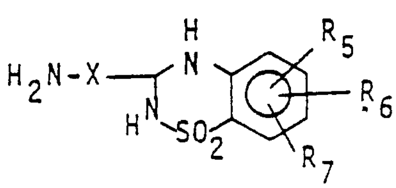

(XI)

unterwirft, wobei die Reste $R_1$ bis $R_9$, X, X', Y, Z, und der Index n die obige Bedeutung haben, und daß man die nach a) bis e) erhaltenen Produkte gewünschtenfalls, sofern zunächst Säureadditionssalze erhalten werden, in freie Basen oder in Salze anderer Säuren überführt und sofern zunächst freie Basen entstehen, diese gewünschtenfalls in Säureadditionssalze überführt und/oder daß man vor oder nach der vorstehenden Umwandlung gewünschtenfalls eine Racematspaltung oder gegebenenfalls Trennung in diastereomere

Antipodenpaare vornimmt.

Europäisches Patentamt

# EUROPÄISCHER RECHERCHENBERICHT

Nummer der Anmeldung

EP 87 11 3750

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 169 495 (BOEHRINGER INGELHEIM)<br>* Ansprüche 1, 5-7 *<br>--- | 1,2,9-11 | C 07 D 285/30<br>C 07 D 285/24<br>C 07 D 417/12<br>A 61 K 31/54 |
| A | DE-A-2 757 922 (BAYER AG)<br>* Ansprüche 1, 2 5 *<br>--- | 1,10,11 | |
| A | US-A-3 287 215 (J.E. ROBERTSON et al.)<br>* Anspruch 1; Spalte 2, Zeilen 4-17 *<br>--- | 1,11 | |
| A | US-A-4 536 501 (J.E. SUNDEEN et al.)<br>* Anspruch 1, Zusammenfassung *<br>--- | 1,2,10 | |
| A | PATENT ABSTRACTS OF JAPAN, Band 9, Nr. 205 (C-299)[1928], 22. August 1985; & JP - A - 60 72868 (FUJISAWA YAKUHIN KOGYO K.K.) 24.04.1985<br>----- | 11 | |

| RECHERCHIERTE SACHGEBIETE (Int. Cl.4) |
|---|
| C 07 D 285/00<br>C 07 D 417/12 |

Der vorliegende Recherchenbericht wurde für alle Patentansprüche erstellt

| Recherchenort | Abschlußdatum der Recherche | Prüfer |
|---|---|---|
| BERLIN | 16-12-1987 | HASS C V F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)